# EUROPEAN PATENT APPLICATION

(11) **EP 4 241 671 A1**
(43) Date of publication of application: **13.09.2023**
(21) Application number: 21902125.0
(22) Date of filing: 13.09.2021
(51) Int. Cl.: A61B 5/055

(54) **TUNING AND MATCHING APPARATUS AND METHOD, MAGNETIC RESONANCE IMAGING DEVICE, AND DISPLAY INTERACTION APPARATUS**

(30) Priority: 07.12.2020 CN 202022906917 U; 07.12.2020 CN 202011417382; 25.08.2021 CN 202110983393
(71) Applicant: Wuhan United Imaging Life Science Instrument Co., Ltd, Wuhan, Hubei 430206 (CN)
(72) Inventor: XU, Changbin, Wuhan, Hubei 430206 (CN); CHEN, Wensong, Wuhan, Hubei 430206 (CN); LI, Sheng, Wuhan, Hubei 430206 (CN)
(74) Representative: Wang, Bo
(86) International application number: PCT/CN2021/117968
(87) International publication number: WO 2022/121417

(57) **Abstract**

Disclosed is tuning and matching devices and methods, magnetic resonance imaging (MRI) equipment, and display interaction devices. The MRI equipment may include a transmitting coil. The tuning and matching control device may be connected with the transmitting coil and configured to perform tuning and matching on the transmitting coil. The display interaction device may be in a scanning room. The tuning and matching method may include: first obtaining curve information of a tuning curve, if determining that the curve information of the tuning curve to be displayed is not within a set information range, adjusting layout information of the tuning curve to be displayed in a display interface, and displaying the adjusted tuning curve in the display interface. According to the method for adjusting display of the tuning curve, the layout information of the tuning curve in the display interface can be adjusted according to the curve information of the tuning curve, thereby making the tuning curve in the display interface clearer and more intuitive, and facilitating users to adjusting ultra-high field MRI equipment to a better state.

## Description

### CROSS REFERENCE TO RELATED APPLICATIONS

This application claims priority to the Chinese Patent No. 202022906917.1, titled "Tuning And Matching Device and Magnetic Resonance Imaging (MRI) Equipment", and filed on December 07, 2020; the Chinese Patent No. 202011417382.X, filed on December 7, 2020, and titled "Tuning and Matching Device and Method, and Magnetic Resonance Imaging (MRI) Equipment", and the Chinese Patent No. 202110983393.2, filed on August 25, 2021, and titled "Method for Adjusting Display of Tuning Curve and Display Interaction Device", the contents of each of which are hereby incorporated by reference in its entirety.

### TECHNICAL FIELD

The present disclosure relates to the technical field of magnetic resonance imaging (MRI), and in particular to tuning and matching devices and methods, MRI equipment, and display interaction devices.

### BACKGROUND

The 9.4T MRI machine serves as an animal experimental research platform, with a magnet diameter potentially reaching 300 mm. The key advantage of the ultra-high field MRI system is its ability to provide a higher signal-to-noise ratio (SNR). The ultra-high field MRI equipment may require tuning and matching of radio frequency (RF) transmitting coils in use. In conventional technical solutions, the ultra-high field MRI equipment may include an operating room, a scanning room, and a cabinet room. A monitor may be placed in the operating room, a display host may be placed in the cabinet room, and tuning and matching knobs may be set on a volume transmitting coil. The volume transmitting coil is arranged at a gantry in the scanning room for tuning and matching. In the current workflow, tuning and matching are initially triggered in the operating room, followed by adjustments using the tuning and matching knobs upon returning to the scanning room. After tuning and matching are completed, the results are viewed on the displayer back in the operating room, leading to inefficiencies in the workflow.

### SUMMARY

One aspect of the present disclosure provides a display interaction device, comprising: a display panel configured to display a tuning curve; a controller configured to: obtain curve information of the tuning curve; and if determining that the curve information of the tuning curve is not within a set information range, adjust layout information of the tuning curve to be displayed in a display interface, and display the adjusted tuning curve in the display interface.

Another aspect of the present disclosure provides a method for adjusting display of a tuning curve, comprising: obtaining curve information of the tuning curve; and if determining that the curve information of the tuning curve is not within a set information range, adjusting layout information of the tuning curve in a display interface, and displaying the adjusted tuning curve in the display interface.

Another aspect of the present disclosure provides a tuning and matching device for MRI equipment. The MRI equipment may include a transmitting coil. The tuning and matching device may include: a tuning and matching control device configured to perform tuning and matching on the transmitting coil by connecting to the transmitting coil; and a display interaction device which is arranged in a scanning room and configured to display tuning and matching data.

Another aspect of the present disclosure provides a tuning and matching device for MRI equipment. The MRI equipment may include a gantry. The tuning and matching device may include: a display interaction device including a display panel which is arranged on the gantry and configured to display tuning and matching data.

Another aspect of the present disclosure provides a tuning and matching device for MRI equipment. The tuning and matching device may include a display interaction device described in any one of the above.

Another aspect of the present disclosure provides a tuning and matching method for MRI equipment. The tuning and matching method may perform tuning and matching on a transmitting coil using any tuning and matching device described above.

Another aspect of the present disclosure provides a tuning and matching method for MRI equipment. The tuning and matching method may include any method for adjusting display of the tuning curve described above.

Another aspect of the present disclosure provides MRI equipment, including any tuning and matching device described above.

Another aspect of the present disclosure provides MRI equipment, including a gantry. The MRI equipment may further include: a display panel which is arranged on the gantry and configured to display a tuning curve.

Another aspect of the present disclosure provides computer equipment, including a storage and a processor. The storage may store computer programs, and when executing the computer programs, the processor may implement operations of any method for adjusting display of the tuning curve described above.

Another aspect of the present disclosure provides a non-transitory computer-readable storage medium storing computer programs that executable by computer equipment. When the computer programs are run on the computer equipment, the operations of any method for adjusting display of the tuning curve may be implemented by the computer equipment.

### BRIEF DESCRIPTION OF THE DRAWINGS

FIG. 1 is a schematic structural diagram illustrating an MRI system according to the prior art;
FIG. 2 is a schematic diagram illustrating a structural connection relationship of a tuning and matching device for MRI equipment according to some embodiments of the present disclosure;
FIG. 3 is a schematic diagram illustrating a display panel of a tuning and matching device for MRI equipment installed on a gantry housing according to some embodiments of the present disclosure;
FIG. 4 is a schematic diagram illustrating a structural connection relationship of a tuning and matching device for MRI equipment according to some embodiments of the present disclosure;
FIG. 5 is a schematic structural diagram illustrating a tuning and matching device for MRI equipment according to some embodiments of the present disclosure;
FIG. 6 is a schematic display diagram illustrating a display panel according to some embodiments of the present disclosure;
FIG. 7 is a schematic diagram illustrating functional segmentation of a display panel according to some embodiments of the present disclosure;
FIG. 8 is a flowchart illustrating a method for adjusting display of a tuning curve according to some embodiments of the present disclosure;
FIG. 9 is a standard curve diagram according to some embodiments of the present disclosure;
FIG. 10 is a curve diagram in a tuning process according to some embodiments of the present disclosure;
FIG. 11 is a curve diagram in a tuning process according to some embodiments of the present disclosure;
FIG. 12 is a curve diagram in a tuning process according to some embodiments of the present disclosure; and
FIG. 13 is a schematic diagram illustrating adjustment rules according to some embodiments of the present disclosure.

### Labels of the drawing:

11. Transmitting coil; 20. Display interaction device; 21. Control host; 22. Display panel; 23. Data acquisition device; 221. Display area; 222. Operating area; 223. Prompt area; 30. Tuning and matching control device; 31. Adjusting screw rod; 32. Tuning and matching circuit; 100. Gantry; 201. Console; 202. Console host.

### DETAILED DESCRIPTION

In order to more clearly illustrate the technical solutions related to the embodiments of the present disclosure, a brief introduction of the drawings referred to the description of the embodiments is provided below. It should be understood that the specific embodiments described here are intended only to explain the present disclosure, and are not to limit the present disclosure.

The serial numbers assigned to components in the present disclosure, such as "first", "second", etc., are intended only to distinguish the described objects, and do not have any sequence or technical meaning. The "connection" and "connecting" mentioned in the present disclosure all include direct and indirect connection unless otherwise specified. In the description of the present disclosure, it should be understood that the orientation or positional relationship indicated by the terms "upper", "lower", "front", "rear", "left", "right", "vertical", "horizontal", "top", "bottom", "inner", "outer", "clockwise", "counterclockwise", etc. is based on the orientation or positional relationship shown in the drawings, and is for the convenience of describing the present disclosure and simplifying the description only, rather than indicating or implying that the device or element referred to must have a particular orientation, be constructed and operate in a particular orientation, and thus should not be construed as limiting the present disclosure.

In the present disclosure, unless otherwise clearly specified and limited, a first feature being "above" or "below" a second feature means that the first and second features are in direct contact, or that the first and second features are in indirect contact through an intermediary. Moreover, the first feature being "above" and "on" the second feature means that the first feature is directly above or obliquely above the second feature, or simply means that the first feature is higher than the second feature. The first feature being "below" and "beneath" the second feature means that the first feature is directly below or obliquely below the second feature, or simply means that the first feature is lower than the second feature.

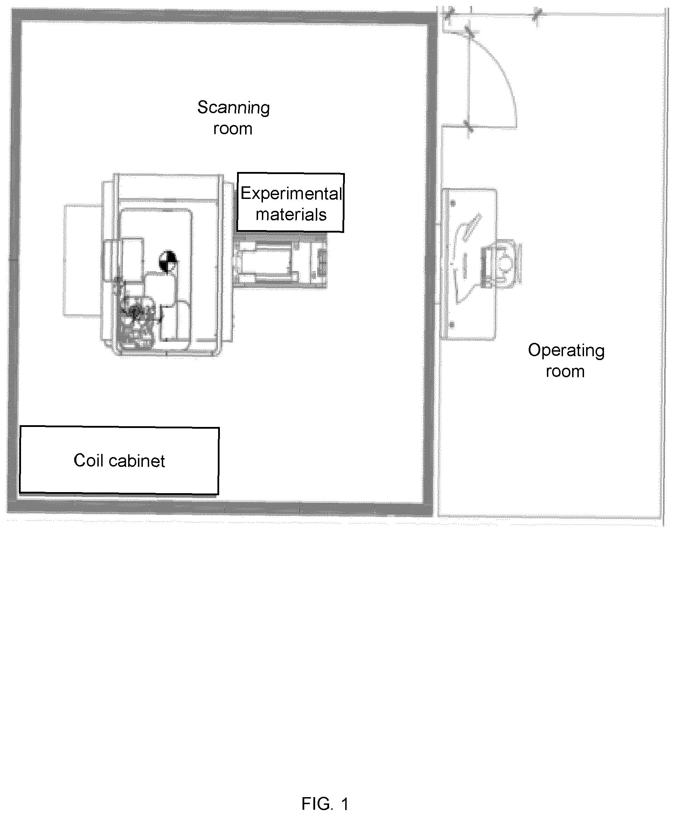

Please refer to FIG. 1. In the prior art, an ultra-high field (e.g., 9.4T) MRI system may include an operating room, a scanning room, and a cabinet room. A gantry of the MRI equipment may be arranged in the scanning room. Magnets, or the like may be arranged on the gantry, to generate an ultra-high magnetic field. A console may be arranged in the operating room. The console may include a console host, a displayer, a keyboard, a mouse, and other interactive control devices for the MRI equipment. Control equipment, power distribution equipment, etc. are arranged in the cabinet room. Before performing magnetic resonance scanning, tuning and matching may be performed on a volume transmitting coil. The volume transmitting coil may be arranged on the gantry. Tuning and matching knobs may be set on the volume transmitting coil. During the tuning and matching process, tuning and matching may be triggered in the operating room first. Subsequently, the adjustments are made by manipulating the tuning and matching knobs upon returning to the scanning room. After tuning and matching are completed, the results can only be viewed on the displayer back in the operating room, and a status of tuning and matching may not be known in the scanning room. If first tuning and matching do not meet the requirements, it is necessary to repeatedly travel between the scanning room and the operating room for confirmation, resulting in low in work efficiency.

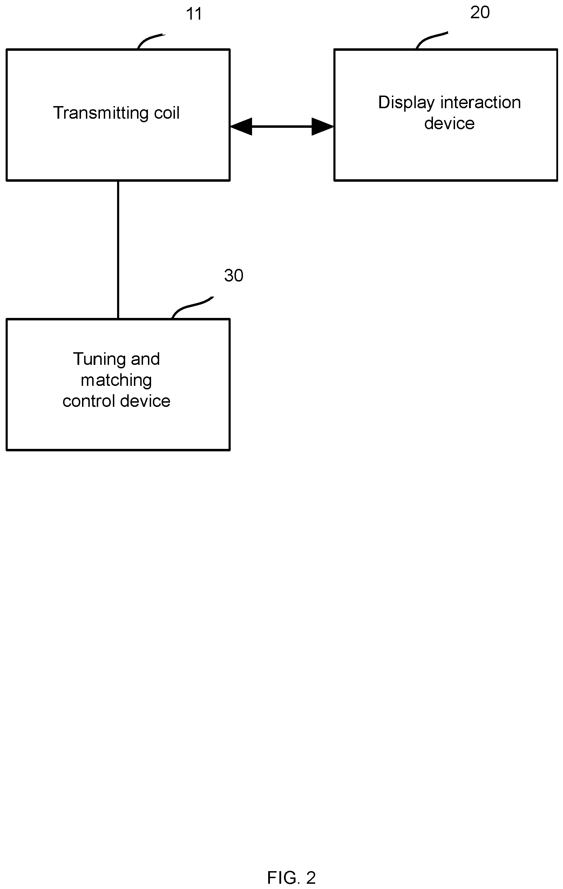

Referring to FIG. 2, some embodiments of the present disclosure provide a tuning and matching device for MRI equipment. In one embodiment, the MRI equipment may be ultra-high field MRI equipment for animals, e.g., a 9.4T MRI equipment for animals. The MRI equipment may include a transmitting coil 11. The tuning and matching device may include a display interaction device 20 and a tuning and matching control device 30.

In some embodiments of the present disclosure, the display interaction device 20 may be arranged in the scanning room. The display interaction device 20 may be connected to the transmitting coil 11, and may control the transmitting coil 11 to transmit test RF pulses or stop transmitting the test RF pulses. It can be understood that the test RF pulses may also be controlled by other internal control devices connected to the display interaction device 20. For example, when the display interaction device 20 is turned on, the transmitting coil 11 may be controlled to start transmitting the test RF pulses, and when the display interaction device 20 is turned off, the transmitting coil 11 may be controlled to stop transmitting the test RF pulses. The tuning and matching control device 30 may be connected to the transmitting coil 11, and may be configured to perform tuning and matching on the transmitting coil 11 when the transmitting coil 11 transmits the test RF pulses. The display interaction device 20 may be configured to display tuning and matching data.

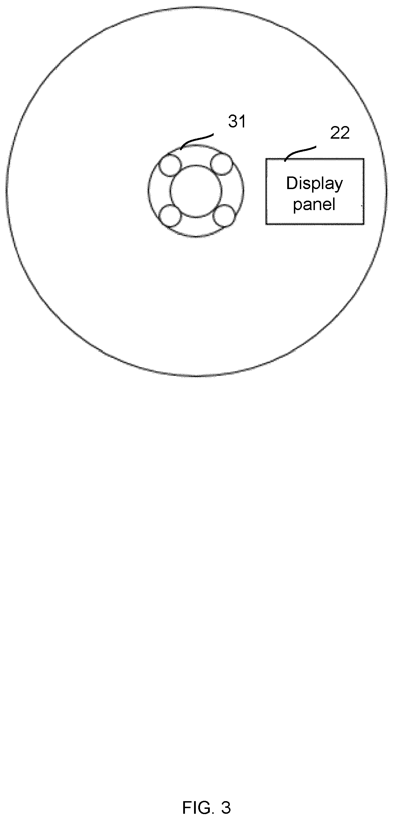

Referring to FIG. 3, in some embodiments, the display interaction device 20 may be arranged near the tuning and matching control device 30. The display interaction device 20 being arranged near the tuning and matching control device 30 means that a display interface (in some embodiments, the display interface is a display panel) of the display interaction device 20 is near the tuning and matching control device 30, so that the staff may view the tuning and matching data when adjusting the tuning and matching control device 30. It can be understood that the tuning and matching control device 30 may include a plurality of components. The display interaction device 20 being arranged near the tuning and matching control device 30 may mean that the display interface of the display interaction device 20 may be arranged near part components (e.g., adjustment components of the tuning and matching control device 30) of the tuning and matching control device 30.

It can be understood that the RF pulses generated by an RF power amplifier may excite the transmitting coil 11 to generate a uniform RF field, thereby exciting an object to be scanned to generate a magnetic resonance signal. A receiving coil may receive the magnetic resonance signal generated from the object to be scanned. The object to be scanned may be an animal or a phantom (e.g., a water phantom). Specifically, the transmitting coil 11 may transmit an RF signal having a same frequency as that of a spin-precession motion to atomic nuclei having the spin-precession motion included in the object to be scanned. Then, the receiving coil may receive a magnetic resonance (MR) signal transmitted from the atomic nuclei included in the object to be scanned.

For example, in order to change the atomic nuclei from a low-energy state to a high-energy state, the transmitting coil 11 may generate and apply an electromagnetic wave signal having an RF corresponding to a type of the atomic nuclei, e.g., an RF signal, to the object. When the electromagnetic wave signal generated by the transmitting coil 11 is applied to the atomic nuclei, the atomic nuclei may change from the low-energy state to the high-energy state. Then, after the transmitting coil 11 stops generating electromagnetic waves, the atomic nuclei to which the electromagnetic waves are applied may change from the high-energy state to the low-energy state, thereby transmitting the electromagnetic waves having a Larmor frequency. In other words, when applying the electromagnetic wave signal to the nuclei is stopped, the energy level of the atomic nuclei may be changed from the high-energy level to the low-energy level, and thus the atomic nuclei may transmit the electromagnetic waves having the Larmor frequency. The receiving coil may receive the electromagnetic wave signal from atomic nuclei included in the object. The received electromagnetic wave signal may be referred to as a free induction decay (FID) signal.

In one embodiment, the transmitting coil 11 may be configured with a volume transmitting coil (VTC) corresponding to an operating frequency of a MR system. The receiving coil may be configured as a loop coil (LC) of a same frequency.

The transmitting coil 11 and the receiving coil may communicate with an external device via wired communication or wireless communication, and may also perform dual tuning and matching communication according to a communication frequency band.

The transmitting coil 11 may be generally a dual-channel coil, which may be used for orthogonal excitation, and generate a uniform RF field; the receiving coil may be a single-channel RF coil, a dual-channel RF coil, or a multi-channel RF coil.

In some embodiments of the present disclosure, a structure of the display interaction device 20 is not specifically limited, as long as the display interaction device 20 can control the transmitting coil 11 to transmit the test RF pulses and display the tuning and matching data.

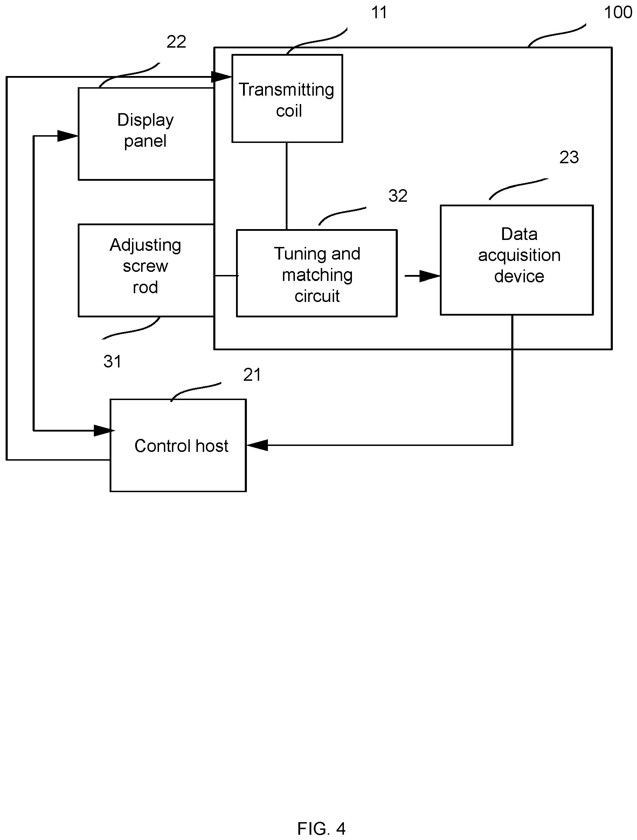

Also referring to FIG. 4, in some embodiments, the display interaction device 20 may include a control host 21, a display panel 22, and a data acquisition device 23. One end of the control host 21 may be in signal connection to the transmitting coil 11. The display panel 22 may be in signal connection to an other end of the control host 21. Tuning and matching start information may be input through the display panel 22. The tuning and matching start information may be sent to the transmitting coil 11 through the control host 21 to control the transmitting coil 11 to transmit the test pulses.

An input end of the data acquisition device 23 may be in signal connection to the tuning and matching control device 30 to obtain the tuning and matching data. An output end of the data acquisition device 23 may be in signal connection to the display interaction device 20 to send the tuning and matching data to the display interaction device 20 for display.

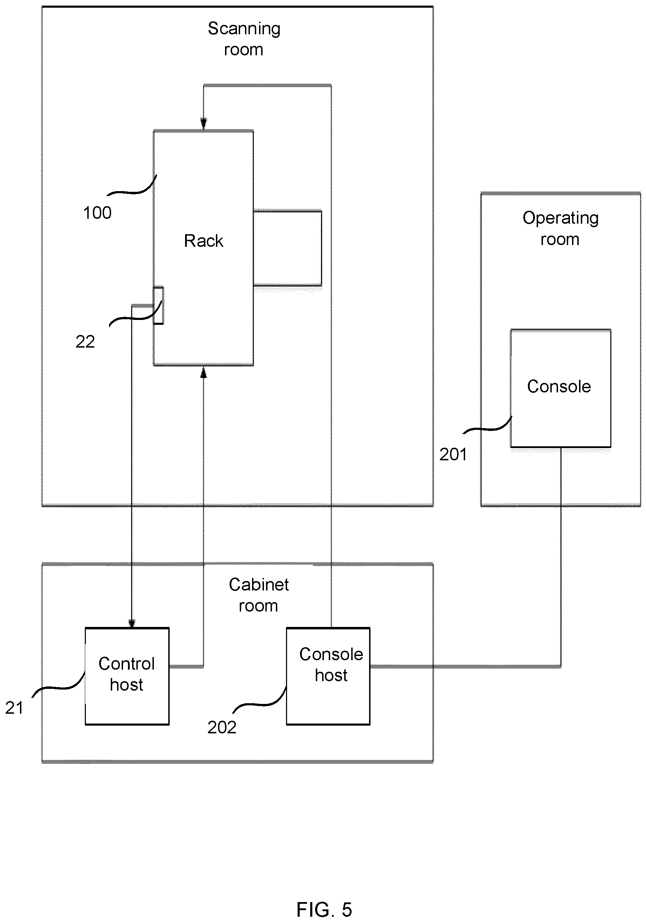

Referring to FIG. 5, a position of the control host 21 is not specifically limited. In some embodiments, the control host 21 may be arranged in the cabinet room. The RF pulses having the Larmor frequency may be pre-stored in the control host 21. The tuning and matching start information may be information for triggering transmitting of the test pulses. When the control host 21 receives the tuning and matching start information, the RF pulses having the Larmor frequency may be provided to the transmitting coil 11 to control the transmitting coil 11 to transmit the test pulses.

It can be understood that in the present disclosure, tuning and matching may be performed using the tuning and matching control device 30, and then the tuning and matching data may be sent to the display interaction device 20 for display, such that there is no need of operations on a console 201 of the operating room during the tuning and matching process. If necessary, the console 201 of the operating room may also be connected to the transmitting coil 11 through the console host 202 to input the tuning and matching start information and display the tuning and matching data in the console 201.

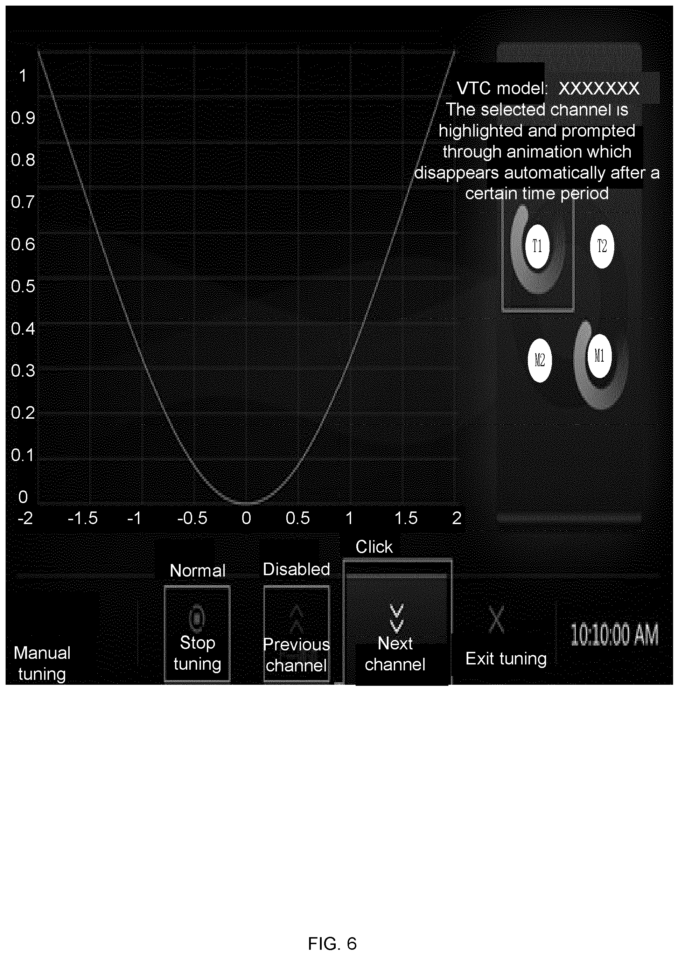

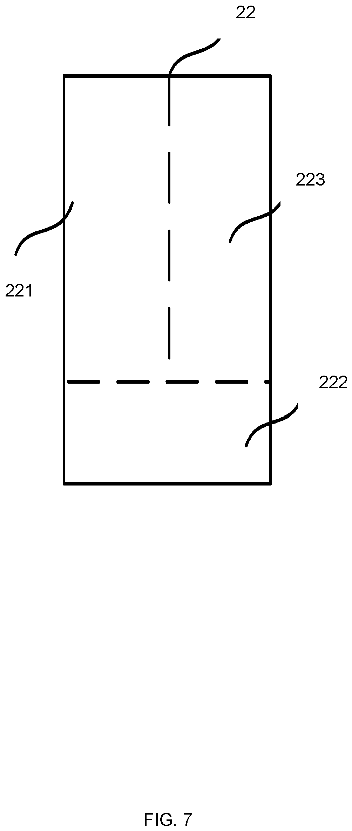

In some embodiments of the present disclosure, referring to FIG. 6 and FIG. 7, a trigger button and a stop button may be set on the display panel 22. A user may send the tuning and matching start information to trigger start of tuning and matching by clicking the trigger button, and the user may stop tuning and matching by clicking the stop button. In one of the embodiments, the display panel 22 may include a plurality of functional areas. The plurality of functional areas may be used in conjunction to implement operation process of inputting the tuning and matching start information or tuning and matching stop information to the transmitting coil 11 and displaying the tuning and matching data.

In some embodiments, the plurality of functional areas may include a display area 221, an operating area 222, and a prompt area 223.

The display area 221 may be configured to display the tuning and matching data. The operating area 222 may be configured to input the tuning and matching start information or the tuning and matching stop information.

Specifically, the display area 221 may be arranged above the display panel 22. An upper left part may display the tuning and matching data (e.g., an RF pulse frequency-reflection coefficient curve, where a vertical axis represents a reflection coefficient, and a horizontal axis represents a relative frequency (according to the Larmor equation w=r*B0, r is the gyromagnetic ratio of hydrogen atoms in water, B0 is a field intensity of a main magnetic field, and w represents a resonance frequency of hydrogen atoms in water under the field intensity of 9.4T). Relevant data during the tuning and matching process may be displayed on the upper left part of the display panel 22 in real time. The status of tuning and matching may be known in real time according to the displayed tuning and matching data, to guide tuning and matching. An upper right part may be the prompt area 223, which may be used to prompt a channel of a transmitting coil to be tuned and matched currently, a screw rod corresponding to the channel of the current transmitting coil for tuning and matching, and/or a screw rod to be adjusted corresponding to the channel of the current transmitting coil for tuning and matching. The prompt area 223 may display a model of the transmitting coil 11 and a position of a knob currently to be operated. That is to say, the user may be instructed to adjust the screw rod 31 of the tuning and matching control device 30 through the position of the knob that to be operated displayed on the upper right part.

A lower part of the display panel 22 may be the operating area 222. The operating area 222 may include a tuning start button, a tuning stop button, a channel selection button, and a tuning exit button. The channel selection button may be located in a channel switching area, and used to switch a tuning channel. These tuning-related buttons may include functions of tuning and matching simultaneously.

It can be understood that the structure of the tuning and matching control device 30 may not be specifically limited, as long as tuning and matching may be performed on the transmitting coil 11. Tuning and matching may be performed on the transmitting coil 11 by the tuning and matching control device 30, and the tuning and matching control device 30 may be electrically connected to the transmitting coil 11.

In one embodiment, the tuning and matching control device 30 may include at least one adjustable capacitor. The tuning and matching control device 30 may perform tuning and matching on the transmitting coil 11 based on whether the RF signal is transmitted through the transmitting coil 11. The tuning and matching control device 30 may adjust a resonant frequency and impedance of the transmitting coil 11. The resonant frequency and the impedance of the transmitting coil 11 may be adjusted respectively by changing capacitance of the adjustable capacitor(s).

The tuning and matching control device 30 may be a manual tuning and matching device. The tuning and matching control device 30 may include adjusting screw rods 31 and a tuning and matching circuit 32. The adjusting screw rods 31 may be connected to the transmitting coil 11 (which is the volume transmitting coil in an optional embodiment), the transmitting coil 11 may be arranged in a gradient coil in the gantry 100, and the adjusting screw rods 31 may extend out of the gantry 100.

In some embodiments of the present disclosure, the display panel 22 may be arranged on the gantry 100. Specifically, the display panel 22 may be arranged on an outer surface of the gantry 100. Besides, the adjusting screw rods 31 and at least one display panel 22 may be located on a same side of the gantry. Optionally, the display panel 22 may be arranged near the adjusting screw rods 31. The display panel 22 being arranged near the adjusting screw rods 31 may mean that the display interface of the display panel 22 may be near the adjusting screw rods 31, such that the staff may also view the tuning and matching data when adjusting the adjusting screw rods 31. It can be understood that there may be a plurality of display panels 22 arranged at different positions of the gantry 100. The plurality of display panels 22 may be arranged on a same side of the gantry 100, or the display panels 22 may be arranged on different sides of the gantry. In one embodiment, the display panel 22 may be arranged on a rear side of the gantry 100. During manual tuning and matching, the transmitting coil 11 may enter a scanning cavity from the rear side of the gantry, and an operator may rotate the adjusting screw rods 31 on the rear side of the gantry. The display panel may also be arranged on a front side of the gantry. The front side of the gantry refers to a side of the gantry where a scanning bed is arranged, and the rear side of the gantry refers to the other side opposite to the front side of the gantry. An input end of the tuning and matching circuit 32 may be connected to the adjusting screw rods 31. An output end of the tuning circuit 32 may be electrically connected to the transmitting coil 11. the adjusting screw rods 31 may be adjusted to adjust output capacitance of the tuning and matching circuit 32, so that the transmitting coil 11 may be tuned and matched. The tuning and matching circuit 32 may include at least two adjustable capacitors. Each tuning and matching channel may correspond to two adjusting screw rods 31. A prompt icon may be correspondingly arranged in the prompt area 223 for a space arrangement position of each adjusting screw rod 31. For example, referring to FIG. 6, cross section positions of the four adjusting screw rods 31 may correspond to T1, M1, T2, and M2 icons of the display interaction device 20 one by one. Optionally, a mark corresponding to the prompt icon may be arranged on each adjusting screw rod 31. That is, it can be understood that a mark "T1" may be set on the adjusting screw rod 31 corresponding to the T1 icon, and a mark "M1" may be set on the adjusting screw rod 31 corresponding to the M1 icon.

The tuning and matching control device 30 may further include at least one switching element. For example, the switching element may be a diode, such as a positive-intrinsic-negative (PIN) diode. The tuning and matching control device 30 may perform tuning and matching on the transmitting coil 11 based on whether the RF signal is transmitted. Tuning and matching may be restored on the transmitting coil 11 via the switched-off tuning and matching control device 30, and tuning and matching may be performed on the transmitting coil 11 via the switched-on tuning and matching control device 30, or vice versa. The tuning and matching control device 30 may be switched through the PIN diode based on transmitting and receiving of the RF signal.

In some embodiments of the present disclosure, the display panel 22 may include an area for displaying a tuning curve. In one embodiment, the tuning and matching data may be the tuning curve (RF pulse frequency-reflection coefficient curve), and the display area 221 may be the area for displaying the tuning curve. After entering a tuning and matching interface, there may be no relevant information in the display area 221. After clicking [Start Tuning] below (after starting tuning, the current button is displayed as Stop Tuning), the system may enter a tuning and matching state, and start to send the tuning and matching data to the display panel 22. The display panel 22 may obtain the data and draw the data in the display area 221. After tuning is started, the upper right area may display an obtained VTC model, and a current channel may be first channel by default. Meanwhile, the T1 and M1 icons in the prompt area 223 may be highlighted (T stands for Tuning, M stands for Match, and T1 and M1 are one set of channels), prompting that the adjusting screw rod T1 and the adjusting screw rod M1 may be rotated currently, and a data curve (tuning curve) of the first channel may be drawn in the upper left area. The adjusting screw rod T1 may be used to tune the resonant frequency of the first channel of the transmitting coil, and the adjusting screw rod M1 may be used to tune impedance matching of the first channel of the transmitting coil. After [Next Channel] is clicked, the T2 and M2 icons in the prompt area 223 may be highlighted, prompting that the adjusting screw rod T2 and the adjusting screw rod M2 may be rotated currently, and a data curve (tuning curve) of a second channel may be drawn in the upper left area. The adjusting screw rod T2 may be used to tune the resonant frequency of the second channel of the transmitting coil, and the adjusting screw rod M2 may be used to tune the impedance matching of the second channel of the transmitting coil. After the tuning is completed, checking may be performed through [Previous Channel] and [Next Channel]. After confirmation, [Exit Tuning] may be clicked to exit the tuning and matching interface.

In some embodiments, in the prompt area, the icon may also be used to prompt an adjusting screw rod 31 corresponding to a current tuning and matching channel of the transmitting coil, specifically, to prompt an rotating adjusting screw rod which is being rotated. For example, when the icons T1 and M1 in the prompt area 223 are highlighted, but the adjusting screw rod 31 which is being rotated is the adjusting screw rod T2 and the adjusting screw rod M2, the icons T2 and M2 in the prompt area 223 may also be lighted to prompt a work personnel to switch to the adjusting screw rod 31 currently being rotated as soon as possible. In some embodiments, as a prompt of abnormal operation, highlight colors or animations of the icons T2 and M2 may be different from those of the icons T1 and M1 for normal operation. For example, the icons T1 and M1 for normal operation may be green in color or clockwise rotating in animation; If the adjusting screw rod T2 or the adjusting screw rod M2 is mis-operated, the icon T2 or M2 may present red or counterclockwise in rotation of animation.

In one embodiment, the tuning and matching control device 30 may be an active circuit. For example, the tuning and matching control device 30 as the active circuit may include at least one PIN diode. A bias circuit may be switched through the PIN diode powered by a direct current (DC) source.

In one embodiment, the tuning and matching control device 30 may be implemented as a passive circuit. For example, the tuning and matching control device 30 as the passive circuit may include at least two PIN diodes connected back-to-back in parallel. The bias circuit may be switched through the two PIN diodes using voltage induced by the RF signal transmitted in an RF signal transmission mode.

Switching requirements of the tuning and matching control device 30 may vary based on elements of tuning and matching control device 30, connectivity between the elements, a size or a type of power supply connected to the bias circuit, or the like.

A data acquisition device 23 may monitor the tuning and matching control device 30. The data acquisition device 23 may monitor one or both of bias voltage and bias current of the tuning and matching control device 30. The bias voltage or the bias current of the tuning and matching control device 30 may be monitored, to determine whether the tuning and matching control device 30 is suitable for operating to perform tuning and matching on the transmitting coil 11. After the data acquisition device acquires one or both of the bias voltage and bias current of the tuning and matching control device 30, data may be converted into a reflection coefficient for display on the display panel 22. For example, after the data acquisition device acquires the bias voltage of the tuning and matching control device 30, a ratio of the bias voltage to input voltage may be converted into the reflection coefficient.

It can be understood that the data acquisition device 23 may also acquire a model of a currently tuned and matched transmitting coil 11 and a currently tuned and matched channel.

The gantry 100, the transmitting coil 11, the display interaction device 20, the tuning and matching control device 30, and the data acquisition device 23 may be connected to each other in a wired or wireless manner, and when they are connected in a wireless manner, the MRI equipment may also include equipment for synchronizing a clock signal among them. Communication between the gantry 100, transmitting coil 11, the display interaction device 20, the tuning and matching control device 30, and the data acquisition device 23 may be implemented through a high speed digital interface (e.g., low voltage differential signaling (LVDS)), asynchronous serial communication (e.g., universal asynchronous receiver/transmitter (UART)), a low-latency network protocol (e.g., error synchronous serial communication or controller area network (CAN)), optical communication, or any other communication methods known to those having ordinary skills in the art.

The MRI equipment may also include a communication interface. The communication interface may be connected to at least one of the gantry 100 in FIG. 5, the display interaction device 20, the tuning and matching control device 30, and the data acquisition device 23.

The communication interface may send data to and receive data from a server connected through a picture archiving and communication system (PACS) or another medical device, and perform data communication according to a digital imaging and communications in medicine (DICOM) standard.

The communication interface may communicate with the server, the medical device, or a portable device by the network in a wired or wireless manner.

Furthermore, the communication interface may send information about failure of the MRI device or about quality of medical images to the user via the network, and receive feedback about the information from the user.

The communication interface may include at least one component capable of communicating with an external device. For example, the communication interface may include a local area communication interface, a wired communication interface, and a wireless communication interface.

The local area communication interface refers to an interface for performing local area communication with a device within a predetermined distance. Examples of local area communication techniques according to exemplary embodiments of the present disclosure may include, but are not limited to, wireless local area network (LAN), Wi-Fi, Bluetooth^{™}, ZigBee^{™}, Wi-Fi Direct (WFD), ultra-wideband (UWB), infrared data association (IrDA), Bluetooth low energy (BLE), and near field communication (NFC).

The wired communication interface refers to an interface for performing communication using an electrical signal or an optical signal. Examples of wired communication techniques according to exemplary embodiments of the present disclosure may include wired communication techniques using twisted pair cables, coaxial cables, and optical fiber cables, and other known wired communication techniques.

The wireless communication interface may transmit a wireless signal to and receive the wireless signal from at least one of a base station, the external device, and the server in the mobile communication network. The wireless signal may be a voice call signal, a video call signal, or data in any one of various formats according to transmitting and receiving of text/multimedia messages.

When the MRI equipment is tuned and matched, tuning and matching may be performed using the tuning and matching control device 30, and then the tuning and matching data may be sent to the display interaction device 20 for display. During the tuning and matching process, operation on the console 201 in the operating room may not required, which may reduce the complexity of the tuning and matching process and improve work efficiency. Besides, as the display interaction device 20 is arranged in the scanning room, the tuning and matching data displayed on the display interaction device 20 may be viewed in real time, making the tuning and matching more accurate.

Based on the same inventive concept, the present disclosure provides a tuning and matching method for MRI equipment. The tuning and matching method may perform tuning and matching on a transmitting coil using the tuning and matching device described in any one of the embodiments.

The tuning and matching method may include controlling the transmitting coil 11 to transmit test pulses by using the display interaction device 20; and when the transmitting coil 11 transmits the test pulses, tuning and matching may be performed on the transmitting coil 11 by using the tuning and matching control device 30, and tuning and matching data may be displayed by using the display interaction device 20.

It can be understood that in some embodiments of the present disclosure, tuning and matching may be performed using the tuning and matching control device 30, and then the tuning and matching data may be sent to the display interaction device 20 for display. During the tuning and matching process, operation on the console 201 in the operating room may not be required, which can reduce the complexity of the tuning and matching process and improve work efficiency. If necessary, the console 201 in the operating room may also be respectively connected to the transmitting coil 11 and the receiving coil through the console host 202 to input the tuning and matching start information and display the tuning and matching data in the console 201.

Before scanning an animal body, the transmitting coil 11 may be installed and fixed at a center position of an aperture of a gradient coil. Meanwhile, at a rear end of the magnet, the cross section positions of the four adjusting screw rods 31 of the transmitting coil and the icons T1, M1, T2 and M2 of the display interaction device 20 may correspond one by one. At this time, the prompt area 223 may automatically display the identified model of the transmitting coil 11. If the tuning and matching interface is triggered, a reflection coefficient curve displayed in the display area 221 of the display panel may represent the resonant frequency and the impedance matching of each channel when the transmitting coil is no-load; then the installed and fixed animal body and the receiving coil may be located to the center position of the transmitting coil, the reflection coefficient curve of the display area 221 may deviate from a no-load trajectory as the animal is pushed in, and finally tuning and matching may be performed: if the adjusting screw rod T1 and the adjusting screw rod M1 are rotated, the icons T1 and M1 in the prompt area 223 may be highlighted, and the display area 221 may display the change of the reflection coefficient curve (tuning curve) of the first channel in real time; if the adjusting screw rod T2 and the adjusting screw rod M2 are rotated, the icons T2 and M2 in the prompt area 223 may be highlighted, and the display area may display the change of the reflection coefficient curve (tuning curve) of the second channel in real time. In this way, the first channel and the second channel may be tuned and matched until a lowest point of the reflection coefficient curve of each channel moves to a set standard area (e.g., ±0.2 on the horizontal axis and within 0.1 on the vertical axis), at such condition, tuning and matching may be ended, Click Stop Tuning and exit.

Based on the same inventive concept, the present disclosure provides MRI equipment, including the tuning and matching device described in any one of the above embodiments.

Based on the same inventive concept, the present disclosure provides a tuning and matching device for MRI equipment. The MRI equipment may include a gantry. The tuning and matching device may include a display interaction device. The display interaction device may include a display panel which is arranged on the gantry and configured to display tuning and matching data. In one embodiment, the display panel may be arranged on a rear side of the gantry. A front side of the gantry may be a side on which a scanning bed is arranged, and the rear side of the gantry may be the other side opposite to the front side of the gantry.

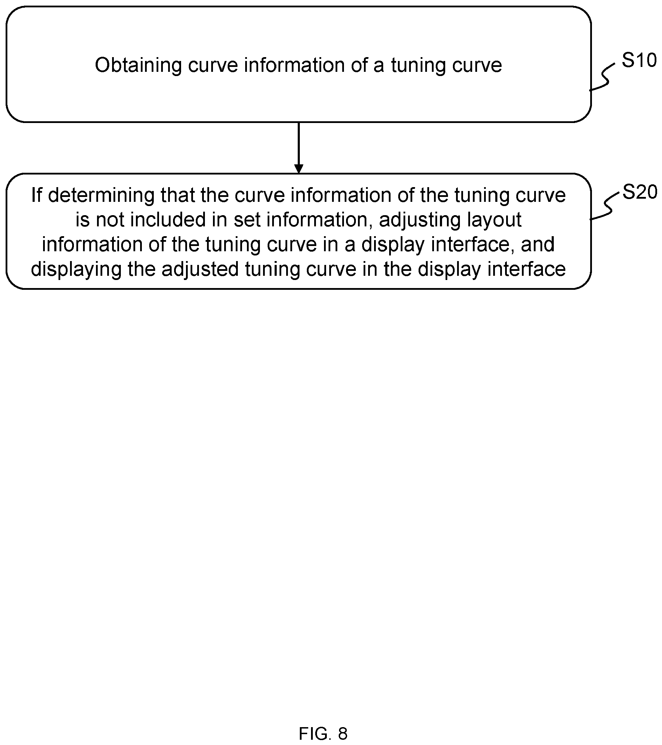

In some embodiments of the present disclosure, referring to FIG. 8, a method for adjusting display of a tuning curve is provided, including:
S10, obtaining curve information of a tuning curve; and
S20, if determining that the curve information of the tuning curve is not within a set information range (also referred to as set information), adjusting layout information of the tuning curve in a display interface, and displaying the adjusted tuning curve in the display interface.

It can be understood that, in S10, the curve information of the tuning curve to be displayed may be obtained by a controller or computer equipment or by a user intuitively with eyes. In one of the embodiments, the curve information of the tuning curve may include position information or shape information.

In S20, the adjustment may be performed only when it is determined that the curve information of the tuning curve to be displayed in the display interface is not within the set information range, which may avoid reducing the user experience caused by real-time change of the curve due to real-time adjustment during the tuning process, thereby guaranteeing user experience and better display of the tuning curve.

The set information may include a standard shape of the curve and a set interval of the curve. It can be understood that, a manner of adjusting the layout information of the tuning curve to be displayed in the display interface may be adjusting a coordinate range corresponding to the tuning curve in the display interface. Generally, an ordinate of the tuning curve may be a reflection coefficient, a value of which may be fixed within a range of 0-1. An abscissa of the tuning curve may be a transmitting frequency. Adjusting a coordinate range corresponding to the tuning curve in the display interface may be adjusting the value range of the abscissa of the tuning curve. In one of the embodiments, the layout information of the tuning curve to be displayed in the display interface may be adjusted manually or automatically. Automatically adjusting the layout information of the tuning curve to be displayed in the display interface requires no user operation, and may cover most application scenarios. Manually adjusting the layout information of the tuning curve to be displayed in the display interface may provide space for the user to operate, and may also cover some scenarios that cannot be satisfied by automatic adjustment. During the tuning process, the curve may be biased to one side, which may cause the main parts not to be displayed or displayed incompletely. In some cases, the automatic adjustment may not be realized or the effect may be still unsatisfactory to the user. The user may switch the automatic adjustment to manual adjustment and adjust the curve using the manual adjustment.

In this embodiment, by using the method for adjusting display of the tuning curve provided in the present disclosure, the layout information of the tuning curve in the display interface may be adjusted according to the curve information of the tuning curve, thereby making the tuning curve in the display interface clearer and more intuitive, and facilitating the user to adjust the ultra-high field MRI equipment to a better state.

In one of the embodiments, before the S10, the method may further include:
obtaining a control instruction for adjusting display of the tuning curve;
if the control instruction is an automatic trigger control instruction, the curve information of the tuning curve being the curve information of the tuning curve to be displayed; and
if the control instruction is a manual trigger control instruction, the curve information of the tuning curve being the curve information of the tuning curve in the display interface.

It should be noted that the automatic trigger control instruction may be automatically triggered by the controller or the computer equipment. The automatic trigger control instruction may also be triggered through a set automatic trigger window. When the user clicks the automatic trigger window, a mode of automatically adjusting the tuning curve to be displayed may be switched on. The manual trigger control instruction may be triggered through a set manual trigger window. When the user clicks the manual trigger window, a mode of manually adjusting the tuning curve to be displayed may be switched on. In one embodiment, the automatic trigger window and the manual trigger window may be adjustment switching keys, and switching between the automatic adjustment and the manual adjustment may be realized by clicking the adjustment switching keys.

In one of the embodiments, S20 may further include:
if it is determined that the shape of the tuning curve in the display interface is not a standard shape,
adjusting a range of the abscissa of the tuning curve to be displayed in the display interface, and displaying the adjusted tuning curve in the display interface.

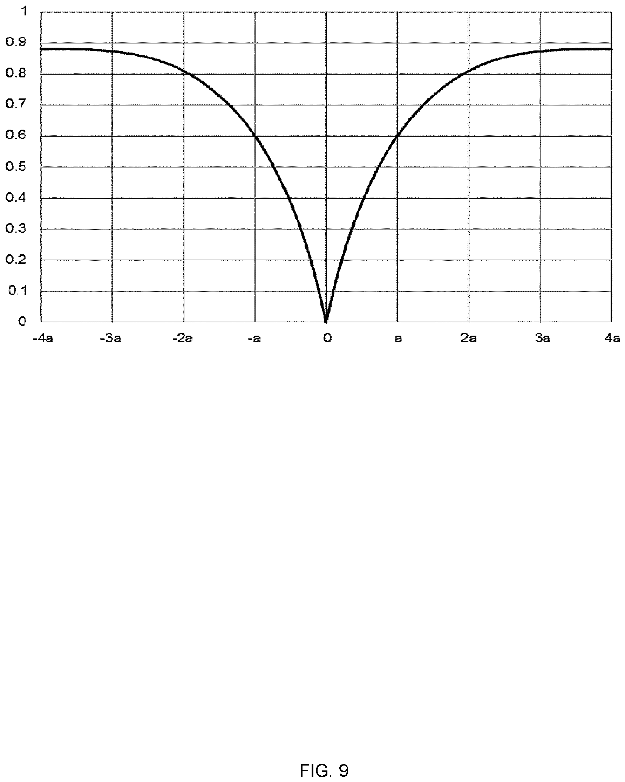

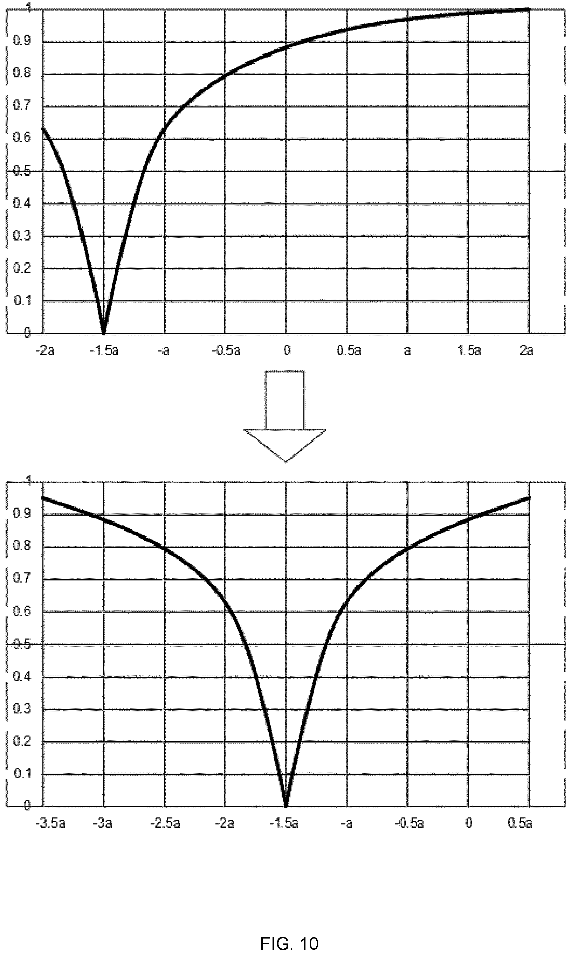

It should be noted that the tuning curve of the standard shape may be understood as the tuning curve when a best state of the equipment can be achieved. As shown in FIG. 9, the embodiment provides a tuning curve of a standard shape. This curve may be displayed completely and bilaterally symmetrical. During the tuning process, the curve may deviate to one side, as shown the upper curve in FIG. 10, the curve may deviate to the left, resulting in incomplete display of an image on a left side. This shape of the tuning curve may be considered as not the standard shape, and the tuning curve may be further tuned. Optionally, the manual adjustment may be adopted. In one embodiment, as shown in FIG. 10, dotted line boxes on left and right sides of the display interface may hide entrances provided for the user to perform the manual adjustment. Taking the right side as an example, when the user clicks an upper dotted line box, a positive data range of the abscissa may be increased, and when the user clicks a lower dotted line box, the positive data range of the abscissa may be decreased. In another embodiment, when the user clicks the right dotted line box, the positive data range of the abscissa may be increased, and when the user clicks the left dotted line box, the positive data range of the abscissa may be decreased. In another embodiment, an abscissa adjustment button may be set in the operating area 222, which specifically may include an abscissa positive data increase button, an abscissa positive data decrease button, an abscissa negative data increase button, and an abscissa negative data decrease button. Optionally, the automatic adjustment may be automatically disabled when the user performs the manual adjustment.

In one of the embodiments, S20 may include:
if it is determined that a position of the tuning curve in the display interface is not within a set interval,
adjusting the range of the abscissa of the tuning curve to be displayed in the display interface, and displaying the adjusted tuning curve in the display interface.

It should be noted that the set interval may be set according to user requirements. In this embodiment, whether the standard curve is clearly displayed may be determined according to whether the position of the tuning curve is within the set interval, which may further be used as a basis for determining whether the tuning curve needs to be adjusted.

In one of the embodiments, before S20, the method may include: obtaining a first distribution interval of the tuning curve to be displayed along a first direction according to the position information of the tuning curve in the display interface; and if the first distribution interval is within the first set interval, determining that the curve information of the tuning curve in the display interface may not be included in the set information.

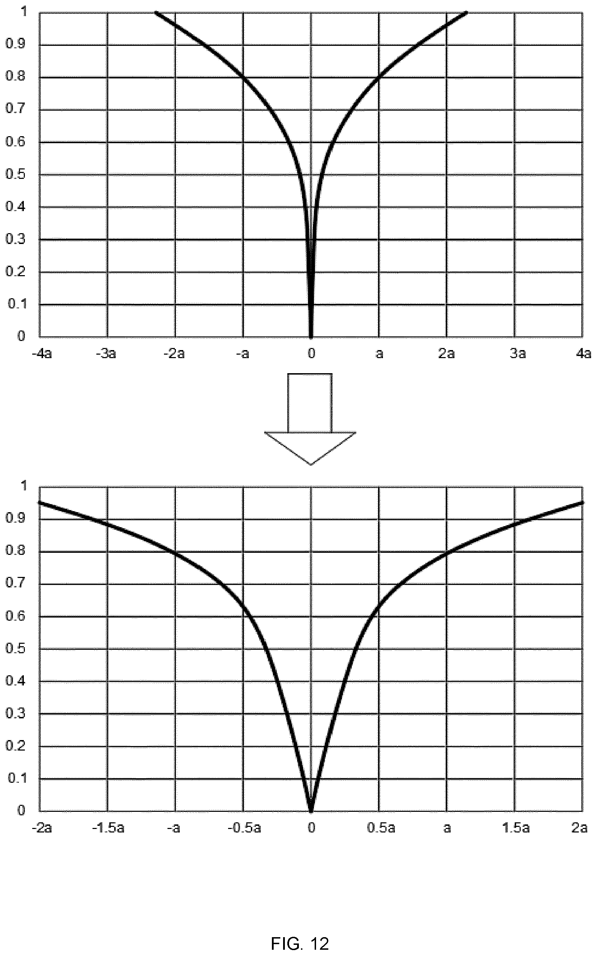

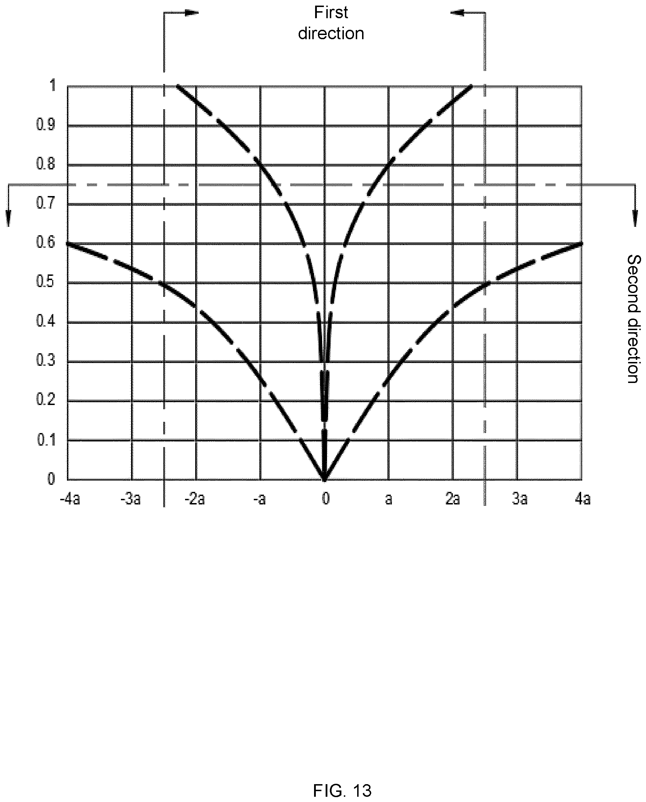

As shown in FIG. 13, the first direction may be a direction extending parallel to the abscissa. The first set interval may be within a range of (-2.5a, 2.5a). Taking FIG. 12 as an example, an upper diagram of FIG. 12 represents that the first distribution interval of the tuning curve along the first direction may be within a range of (-2.5a, 2.5a). The whole tuning curve is in a middle of a grid, but part of tuning curve overlaps and cannot be seen clearly. Then the current tuning curve may be identified as needing further adjustment. In one embodiment, the range of the abscissa may be reduced by adjusting the layout of the display interface using the controller, and the curve may be extended in a horizontal direction, thereby obtaining the tuning curve shown in the lower diagram of FIG. 12. This diagram more clearly shows an original overlapping part. In another embodiment, the range of the abscissa may be reduced by manually adjusting the layout of the display interface, and the curve may be extended in the horizontal direction, thereby more clearly displaying the original overlapping part.

In another embodiment, before S20, the method may include: obtaining a second distribution interval of the tuning curve to be displayed along a second direction according to the position information of the tuning curve in the display interface, the second direction being perpendicular to the first direction; and if the second distribution interval is within the second set interval, determining that the curve information of the tuning curve in the display interface is not included in the set information.

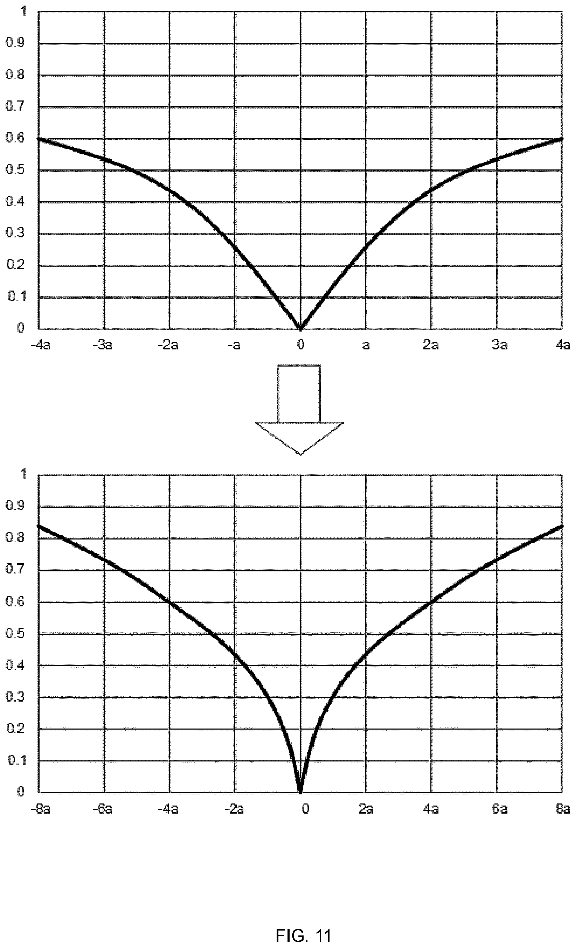

As shown in FIG. 13, the second direction may be a direction extending parallel to the ordinate. The second set interval may be within a range of (0, 0.75). Taking FIG. 11 as an example, an upper diagram of FIG. 11 represents that the second distribution interval of the tuning curve along the second direction may be within a range of (0, 0.75). The whole tuning curve may be located in a lower half part of the coordinate grid. Then the current tuning curve may be identified as requiring further adjustment. In one embodiment, the range of the abscissa may be extended by adjusting the display interface using the controller, and the curve may be extended in the vertical direction, thereby obtaining the tuning curve shown in the lower diagram of FIG. 11. In another embodiment, the curve may be extended in the vertical direction by extending the range of the abscissa by manually adjusting the layout of the display interface.

Based on the same inventive concept, some embodiments of the present disclosure further provide a display interaction device, including a display panel and a controller.

The display panel may be configured to display a tuning curve. The controller may be configured to:
obtain curve information of the tuning curve; and
if it is determined that the curve information of the tuning curve to be displayed is not within a set information range, adjust layout information of the tuning curve to be displayed in a display interface, and display the adjusted tuning curve in the display interface.

Some embodiments of the present disclosure further provide a tuning and matching method for MRI equipment. The tuning and matching method may perform tuning and matching on a transmitting coil using a tuning and matching device. The tuning and matching device may include a display panel arranged on a rear side of a gantry of the MRI equipment. The tuning and matching method may include: controlling the transmitting coil to transmit test pulses using the display panel; and displaying tuning and matching data using the display panel. In some embodiments, the tuning and matching method may include operations of the method for adjusting display of the tuning curve described in any of the above embodiments.

The present disclosure provides computer equipment, including a storage and a processor. The storage may store computer programs. When executing the computer programs, the processor may implement the method for adjusting display of the tuning curve described in any one of the above embodiments.

As a non-transitory computer-readable storage medium, the storage may be configured to store software programs, computer-executable programs, and modules, such as program instructions/modules corresponding to the method for adjusting display of the tuning curve in the embodiments of the present disclosure. The processor may execute, i.e., implement the method, various functional applications, and data processing of the equipment by running the software programs, the instructions and the modules stored in the storage.

The storage may mainly include a program storage area and a data storage area. The program storage area may store an operating system and an application program required by at least one function. The data storage area may store data, or the like, created according to the use of a terminal. In addition, the storage may include highspeed random access memory, and may also include non-volatile memory, such as at least one magnetic disk storage device, flash memory device, or other non-volatile solid-state storage devices. In some examples, the storage may further include a storage located remotely from the processor. The remote storage may be connected to the equipment via networks. Examples of the networks may include, but are not limited to, the Internet, intranets, local area networks, mobile communication networks, or combinations thereof.

The technical features of the embodiments may be combined arbitrarily. To make the description concise, not all possible combinations of the technical features in the embodiments are described. However, as long as there is no contradiction in the combinations of these technical features, these should be considered as within the scope of the present disclosure.

The embodiments are only preferred embodiments of the present disclosure, and are not intended to limit the present disclosure. Any modifications, equivalent replacements and improvements made within the spirit and principles of the present disclosure should be included in the protection scope of the present disclosure. The above embodiments are only the preferred embodiments of the present disclosure. It should be noted that for those having ordinary skills in the art, some improvements and modifications may be made without departing from the technical principles of the present disclosure. These improvements and modifications should also fall within the protection scope of the present disclosure.

## Claims

1. A display interaction device, comprising:
a display panel configured to display a tuning curve; and
a controller configured to:
obtain curve information of the tuning curve; and
if determining that the curve information of the tuning curve is not within a set information range, adjust layout information of the tuning curve in a display interface, and display the adjusted tuning curve in the display interface.

2. A tuning and matching device for magnetic resonance imaging (MRI) equipment, wherein the MRI equipment includes a transmitting coil, and the tuning and matching device includes:
a tuning and matching control device which is connected to the transmitting coil and configured to perform tuning and matching on the transmitting coil; and
a display interaction device which is arranged in a scanning room and configured to display tuning and matching data.

3. The tuning and matching device for the MRI equipment of claim 2, wherein the display interaction device is further configured to start or stop tuning and matching.

4. The tuning and matching device for the MRI equipment of claim 2, wherein the MRI equipment further includes a gantry, the display interaction device includes a display panel, and the display panel is arranged on the gantry.

5. The tuning and matching device for the MRI equipment of claim 4, wherein the display panel includes a plurality of functional areas, and the plurality of functional areas include:
a display area configured to display the tuning and matching data; and
an operating area configured to input tuning and matching start information or tuning and matching stop information.

6. The tuning and matching device for the MRI equipment of claim 5, wherein the operating area further includes: a channel switching area configured to switch channels of the transmitting coil for tuning and matching.

7. The tuning and matching device for the MRI equipment of claim 6, wherein the tuning and matching control device includes: adjusting screw rods, each of the channels corresponds to two adjusting screw rods, and the two adjusting screw rods adjust impedance matching and a resonant frequency of the channel of the transmitting coil, respectively; and
the plurality of functional areas further include: a prompt area configured to prompt an adjusting screw rod which is currently rotated, and/or prompt a correct adjusting screw rod corresponding to a channel of the transmitting coil currently being tuned and matched.

8. The tuning and matching device for the MRI equipment of claim 7, wherein a prompt icon corresponding to a spatial arrangement position of each adjusting screw rod is arranged in the prompt area; and a mark corresponding to the prompt icon is arranged on each adjusting screw rod.

9. The tuning and matching device for the MRI equipment of claim 8, wherein the prompt icon prompts a channel currently to be tuned and matched by highlighting or animation.

10. A tuning and matching device for MRI equipment, wherein the MRI equipment includes a gantry, and the tuning and matching device includes:
a display interaction device including a display panel which is arranged on the gantry and configured to display tuning and matching data.

11. The tuning and matching device for the MRI equipment of claim 10, wherein the display interaction device includes: a controller configured to:
obtain curve information of a tuning curve; and
if determining that the curve information of the tuning curve is not within a set information range, adjust layout information of the tuning curve in a display interface, and display the adjusted tuning curve in the display interface.

12. Magnetic resonance imaging (MRI) equipment, comprising the tuning and matching device of any one of claims 2-11.

13. Magnetic resonance imaging (MRI) equipment, comprising a gantry and a display panel, the display panel being arranged on the gantry and including an area for displaying a tuning curve.

14. A method for adjusting display of a tuning curve, comprising:
obtaining curve information of a tuning curve; and
if determining that the curve information of the tuning curve is not within a set information range, adjusting layout information of the tuning curve in a display interface, and displaying the adjusted tuning curve in the display interface.

15. The method for adjusting display of the tuning curve of claim 14, wherein the curve information of the tuning curve includes position information or shape information.

16. The method for adjusting display of the tuning curve of claim 15, wherein before obtaining the curve information of the tuning curve, the method further includes:
obtaining a control instruction for adjusting display of the tuning curve;
if the control instruction is an automatic trigger control instruction, determining the curve information of the tuning curve as curve information of a tuning curve to be displayed; and
if the control instruction is a manual trigger control instruction, determining the curve information of the tuning curve as curve information of a tuning curve in the display interface.

17. The method for adjusting display of the tuning curve of claim 15, wherein the if determining that the curve information of the tuning curve is not within a set information range, adjusting layout information of the tuning curve in a display interface, and displaying the adjusted tuning curve in the display interface includes:
if determining that a shape of the tuning curve is not a standard shape, adjusting a range of an abscissa of the tuning curve in the display interface, and displaying the adjusted tuning curve in the display interface.

18. The method for adjusting display of the tuning curve of claim 15, wherein the if determining that the curve information of the tuning curve is not within a set information range, adjusting layout information of the tuning curve in a display interface, and displaying the adjusted tuning curve in the display interface includes:
if determining that a position of the tuning curve is not within a set interval,
adjusting a range of an abscissa of the tuning curve in the display interface, and displaying the adjusted tuning curve in the display interface.

19. The method for adjusting display of the tuning curve of claim 18, wherein before the operation of if determining that the curve information of the tuning curve is not within a set information range, adjusting layout information of the tuning curve in a display interface, and displaying the adjusted tuning curve in the display interface, the method further includes:
obtaining a first distribution interval of the tuning curve along a first direction according to position information of the tuning curve; and
if the first distribution interval is within a first set interval, determining that curve information of the tuning curve in the display interface is not included in the set information range.

20. The method for adjusting display of the tuning curve of claim 19, wherein before the operation of if determining that the curve information of the tuning curve is not within a set information range, adjusting layout information of the tuning curve in a display interface, and displaying the adjusted tuning curve in the display interface, the method further includes:
obtaining a second distribution interval of the tuning curve along a second direction according to the position information of the tuning curve, the second direction being perpendicular to the first direction; and
if the second distribution interval is within a second set interval, determining that the curve information of the tuning curve in the display interface is not included in the set information range.

21. A tuning and matching method for magnetic resonance imaging (MRI) equipment, comprising:
performing tuning and matching on a transmitting coil by using a tuning and matching device, wherein the tuning and matching device includes a display panel arranged on a rear side of a gantry of the MRI equipment,
controlling the transmitting coil to transmit test pulses by using the display panel; and
displaying tuning and matching data by using the display panel.

22. The tuning and matching method for the MRI equipment of claim 21, comprising the operations of the method for adjusting display of the tuning curve of any one of claims 14-20.

23. Computer equipment, comprising a storage and a processor, wherein the storage stores computer programs, and when executing the computer programs, the processor implements the method for adjusting display of the tuning curve of any one of claims 14-20.

24. A non-transitory computer-readable storage medium, storing computer programs executable by computer equipment, wherein when the computer programs are run on the computer equipment, the computer equipment executes the method for adjusting display of the tuning curve of any one of claims 14-20.
